# FASCICULE DE BREVET EUROPEEN

(11) **EP 1 474 194 B1**
(45) Date de publication et mention de la délivrance du brevet: **13.04.2011**
(21) Numéro de dépôt: 03739519.1
(22) Date de dépôt: 11.02.2003
(51) Int. Cl.: A61M 5/50

(54) **DISPOSITIF DE SUPPORT DE SECURITE POUR UNE SERINGUE ET ENSEMBLE D UN TEL DISPOSITIF ET D UNE SERINGUE**
SICHERHEITSTRAGVORRICHTUNG FÜR EINE SPRITZE UND EINE SOLCHE VORRICHTUNG UND EINE SPRITZE UMFASSENDE ANORDNUNG
SAFETY SUPPORT DEVICE FOR A SYRINGE AND AN ASSEMBLY COMPRISING ONE SUCH DEVICE AND A SYRINGE

(30) Priorité: 11.02.2002 FR 0201643
(43) Date de publication de la demande: 10.11.2004
(60) Demande divisionnaire: 10184229.2
(73) Titulaire: Sanofi-Aventis, 75013 Paris (FR)
(72) Inventeur: CHEVALIER, Stéphane, 77178 Saint Pathus (FR)
(74) Mandataire: Intès, Didier Gérard André
(86) Numéro de dépôt international: PCT/FR2003/000423
(87) Numéro de publication internationale: WO 2003/068298

(56) Documents cités:
- WO-A-01/85239
- FR-A- 2 801 795
- US-B1- 6 319 234

## Description

La présente invention concerne un dispositif de support de sécurité pour une seringue, comprenant un fourreau de support pour le corps d'une seringue et des moyens de sécurité qui comprennent un fourreau intérieur susceptible de coulisser par rapport au dit fourreau de support, entre une position escamotée d'attente, dans laquelle ledit fourreau intérieur est sensiblement escamoté à l'intérieur dudit fourreau de support, et une position active de protection, dans laquelle ledit fourreau intérieur dépasse dudit fourreau de support.

L'invention concerne également un ensemble constitué d'un tel dispositif et d'une seringue.

Un tel dispositif permet de protéger l'utilisateur contre tout risque de piqûre accidentelle après utilisation de la seringue et donc d'une contamination éventuelle, en entourant l'aiguille de la seringue par le fourreau intérieur, qui est sollicité vers sa position active de protection par des moyens de poussée tels qu'un ressort.

On connaît de tels dispositifs et ensembles qui permettent de protéger l'aiguille par déplacement d'un fourreau intérieur dans un fourreau de support.

Le document FR 2 801 795 divulgue les caractéristiques du préambule de la revendication 1 et décrit un ensemble dans lequel la sortie du fourreau intérieur peut être déclenchée automatiquement par des moyens de poussée (en l'espèce, par des moyens élastiques) pour adopter en fin d'injection, une position active de protection autour de l'aiguille. Cependant, le premier contact du fourreau intérieur avec la chair du patient peut être quelque peu inattendu et surprendre le patient, d'autant que le contact s'effectue de manière dynamique. Par la suite, une légère pression, due à la poussée exercée par les moyens élastiques sur le fourreau intérieur jusqu'à son extraction totale hors du fourreau de support, peut être ressentie par le patient, jusqu'à ce que l'aiguille soit complètement retirée de sa chair. Toutefois, cette pression est statique, donc sans risque contondant pour le patient.

Dans ce type de dispositif, les moyens élastiques sont généralement tarés pour limiter l'intensité de l'appui en provoquant un coulissement délicat du fourreau intérieur hors du fourreau de support qui ne heurte pas la chair du patient. Ainsi, le contact contre la chair du patient se fait plus ou moins délicatement selon la vitesse à laquelle le fourreau intérieur est entraîné hors du fourreau de support, et de manière générale, selon l'intensité de poussée exercée par les moyens élastiques sur le fourreau intérieur.

Cependant, l'impact du fourreau intérieur sur sa peau peut être désagréable pour le patient qui est généralement déjà impressionné par la piqûre.

La présente invention , telle que définie dans les revendications, a pour but de proposer un dispositif de support de sécurité pour seringue dont la sortie en fin d'injection du fourreau intérieur hors du fourreau de support se fait en limitant l'appui entre le fourreau intérieur et la chair du patient.

Ce but est atteint grâce au fait que le fourreau intérieur présente une portion d'extrémité libre formée par un embout souple.

Au contraire des dispositifs connus dans lesquels le patient est amené, dans la zone de la piqûre à encaisser une partie, voire la totalité, de l'énergie libérée par le contact du fourreau intérieur contre sa peau, puisque l'extrémité du fourreau est rigide, le dispositif de la présente invention comporte une portion d'extrémité libre du fourreau intérieur souple qui est capable d'absorber au moins une partie de cette énergie libérée lors de l'appui, et d'amortir ce dernier de sorte qu il s'effectue sensiblement sans heurt.

Avantageusement, le fourreau intérieur comporte une première partie rigide par laquelle le fourreau intérieur coopère avec ledit fourreau de support, et une deuxième partie formée par l'embout souple qui est solidaire de ladite première partie.

La première partie du fourreau intérieur est avantageusement rigide pour éviter tout risque d'arc-boutement lors de son coulissement dans le fourreau de support, ce qui risquerait de gêner sa sortie.

L'embout est avantageusement fixé à la première partie par des moyens choisis parmi le collage, le surmoulage, le soudage ou l'emmanchement en force.

L'embout et la première partie peuvent alors être réalisés en deux pièces avec des matériaux adaptés à la rigidité recherchée.

L'embout peut aussi être fixé à la première partie du fourreau intérieur par des organes de fixation. Dans ce cas, l'embout est avantageusement fixé à ladite première partie par des moyens comprenant des premiers moyens de fixation comportant un organe en saillie et appartenant à l'un des deux éléments constitués par ladite première partie et ledit embout, et des deuxièmes moyens de fixation comportant un organe en retrait appartenant à l'autre de ces deux éléments.

Avantageusement, l'embout présente une extrémité libre amincie et le matériau dudit embout présente une dureté Shore comprise entre 30 Hs et 80 Hs.

Ainsi, l'embout est adapté pour amortir une partie significative de l'énergie qui a pu être accumulée jusqu'au moment de la sortie du fourreau intérieur, en fin d'injection. Le matériau constituant l'embout, ainsi que sa géométrie peuvent être préférentiellement choisis en fonction des moyens de poussée retenus. En particulier, lorsque ces derniers comportent des moyens élastiques de raideur connue, le choix du matériau de l'embout est effectué en fonction de la dureté Shore adéquate pour amortir l'appui contre la peau du patient et pour que l'embout puisse encaisser une partie suffisante de l'énergie accumulée dans les moyens élastiques.

Le dispositif comporte en outre avantageusement des moyens de retenue susceptibles d'adopter une configuration active de retenue dans laquelle ils retiennent le fourreau intérieur en position escamotée d'attente et une configuration inactive dans laquelle ils autorisent la sortie dudit fourreau intérieur sous l'action de moyens de poussée.

Avantageusement, les moyens de retenue comportent des languettes élastiques de retenue et des surfaces de retenue, lesdites languettes ou lesdites surfaces de retenue étant solidaires dudit fourreau intérieur, lesdites languettes de retenue étant retenues sur les surfaces de retenue en configuration active de retenue et étant susceptibles d'être déplacées élastiquement vers leur configuration inactive dans laquelle lesdites languettes de retenue échappent aux dites surfaces de retenue.

L'invention concerne également un ensemble d'un dispositif tel que précédemment décrit et d'une seringue ayant un corps, un piston et une aiguille disposée à une extrémité dudit corps.

Avantageusement, le corps de seringue est maintenu fixement dans le fourreau de support et le piston de la seringue comporte une partie de commande apte à commander les moyens de retenue vers leur configuration inactive.

Le fourreau intérieur et le fourreau de support forment deux tubes concentriques dans lesquels le corps d'une seringue peut être engagé en étant calé par rapport au tube extérieur formé par le fourreau de support. L'utilisateur manipule cet ensemble en saisissant le fourreau de support, par exemple en le calant entre l'index et le majeur, et en poussant le piston de la seringue pour réaliser l'injection, par exemple avec le pouce. Lorsque le piston arrive en fin de course, les moyens de retenue du fourreau intérieur sont désactivés pour permettre la sortie automatique du fourreau intérieur hors du fourreau de support, tandis que le corps de la seringue est maintenu fixement dans ce dernier.

Le fourreau intérieur parvient au contact de la peau du patient, puis il coulisse davantage hors du fourreau de support au fur et à mesure du retrait progressif de l'aiguille hors de la chair du patient, de sorte que l'utilisateur n'a aucun risque de blessure par piqûre avec l'aiguille, puisque cette dernière n'est plus accessible après injection. L'utilisateur ne doit donc prendre aucune disposition particulière quant au positionnement de ses doigts qui ne varie pas, depuis le début de l'injection jusqu'au retrait total de l'aiguille hors de la chair du patient.

Les languettes de retenue des moyens de retenue sont avantageusement situées au voisinage d'une extrémité du fourreau de support opposée à l'aiguille et le piston présente une partie de commande, par exemple sa tête, apte à solliciter lesdites languettes de retenue vers leur configuration inactive.

Ainsi, en fin d'injection, l'utilisateur n'effectue aucun mouvement supplémentaire pour permettre la sortie du fourreau intérieur qui a lieu alors que l'aiguille n'a pas encore été retirée de la chair du patient.

Avantageusement, l'ensemble comporte en outre un capuchon amovible recouvrant l'aiguille de sorte qu'en position escamotée du fourreau intérieur, l'embout du fourreau intérieur entoure sensiblement sans jeu ledit capuchon. Cet embout peut même présenter une légère élasticité radiale lui permettant d'épouser le contour du capuchon.

L'ensemble, en particulier le corps de seringue et l'aiguille, est ainsi protégé de tout risque de contamination extérieure, par exemple bactérienne, depuis son assemblage jusqu'au moment où l'on retire le capuchon, en particulier pour réaliser l'injection.

L'invention sera bien comprise et ses avantages apparaîtront mieux à la lecture de la description détaillée qui suit, de modes de réalisation représentés à titre d'exemples non limitatifs.

La description se réfère aux dessins annexés sur lesquels :
- la figure 1 est une vue en coupe d'un ensemble d'un dispositif de support de sécurité et d'une seringue en position escamotée d'attente,
- la figure 2A est une vue partielle en perspective prise selon la direction II indiquée sur la figure 1,
- la figure 2B est une coupe partielle selon les flèches IIB-IIB de la figure 1,
- la figure 3 est une vue en coupe de l'ensemble de la figure 1 en position active de protection,
- la figure 4 est une vue partielle en coupe de la partie avant du fourreau intérieur selon un autre mode de réalisation, et
- la figure 5 est une vue partielle en coupe de la partie avant du fourreau intérieur selon un autre mode de réalisation.

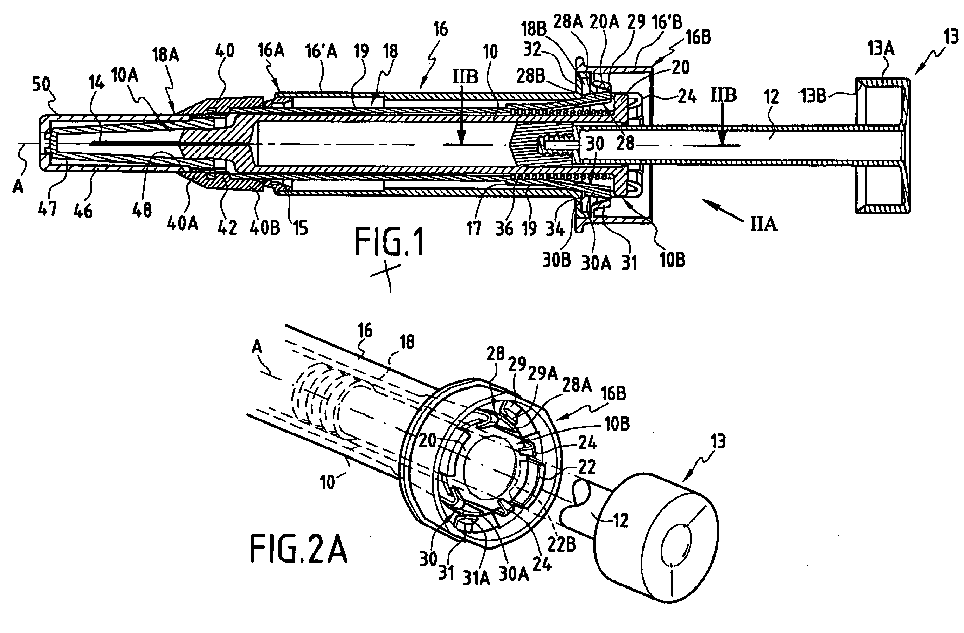

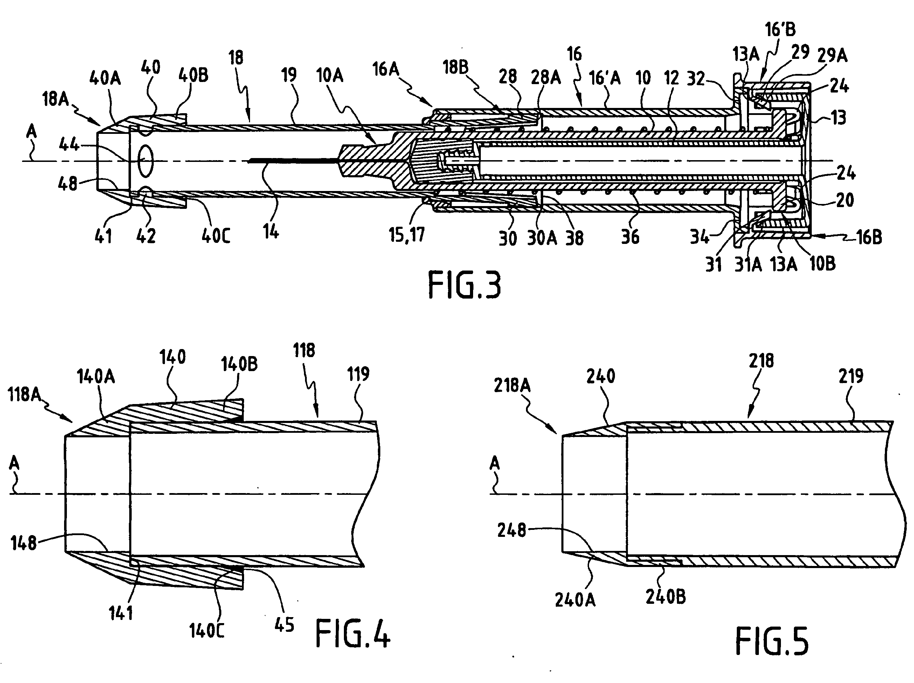

La seringue représentée sur les figures 1 à 4, comporte un corps 10 dans lequel un piston 12 peut coulisser entre une position d'attente d'injection, représentée sur la figure 1, et une position de fin d'injection, représentée sur les figures 3 et 4. A l'opposé du piston, une aiguille d'injection 14 est raccordée au corps 10. Le corps de la seringue 10 est préférentiellement en verre ou en plastique.

Le dispositif de support de sécurité pour cette seringue comporte un fourreau extérieur de support 16 et un fourreau intérieur 18 qui peut coulisser entre une position escamotée d'attente avant injection, représentée sur la figure 1, et une position active de protection après injection, représentée sur les figures 3 et 4. En position escamotée d'attente avant injection, le fourreau intérieur 18 est escamoté dans le fourreau extérieur 16, c'est-à-dire quasiment totalement rentré dans ce dernier, en étant disposé autour du corps 10 de la seringue et en étant retenu par rapport au fourreau extérieur 16 de telle sorte que l'aiguille 14 dépasse au-delà des extrémités avant 16A et 18A respectives des fourreaux intérieur 18 et de support 16.

Ces extrémités avant 16A et 18A sont bien entendu celles qui sont les plus proches du point d'injection, le sens vers l'avant étant le sens F de poussée du piston 12 dans le corps de seringue 10 pendant l'injection.

Le corps de seringue 10 est calé fixement par rapport au fourreau de support 16 par des moyens de calage appartenant à ce fourreau 16 et coopérant avec une collerette radiale 20 que présente l'extrémité arrière 10B du corps de seringue 10 (extrémité opposée à l'aiguille 14). La collerette 20 se trouve alors calée entre une butée comprenant en l'espèce deux épaulements 22B réalisés chacun à partir d'une portion de paroi axiale 22 rigides et des pattes d'encliquetage 24 ménagées à l'intérieur de ce fourreau de support 16 (par exemple, quatre pattes régulièrement réparties sur la périphérie interne de ce dernier). Ces deux portions de paroi axiale 22 sont diamétralement opposées sur le fourreau de support 16.

Lors de la mise en place du corps de seringue 10 dans le dispositif de support de sécurité, les pattes d'encliquetage 24, formées en arrière des épaulements 22B, s'écartent élastiquement pour permettre le passage de la collerette 20 du corps de seringue 10. Ainsi disposée, la collerette 20 est bien calée entre les épaulements 22B (dans le sens F de rentrée du piston) et les pattes d'encliquetage 24 (dans le sens opposé), de sorte que le corps de seringue 10 est maintenu fixement dans le fourreau de support 16 tout au long de l'injection et même pendant la sortie automatique en position active de protection du fourreau intérieur 18.

Comme on le voit plus en détails sur la figure 2A, la partie d'extrémité arrière 18B du fourreau intérieur 18 comporte en outre des languettes de retenue 28 et 30 axiales, dont les portions d'extrémités libres 28A et 30A présentant un épaulement 28B, respectivement 30B qui est respectivement accroché sur des surfaces de retenue 32 et 34 appartenant au fourreau de support 16. En l'espèce, ces surfaces de retenue 32 et 34 sont formées par deux épaulements respectifs ou par des parties d'un épaulement circulaire. En configuration active de retenue, les languettes de retenue 28 et 30 qui sont élastiques, ont une tendance naturelle à s'ouvrir en s'écartant élastiquement vers la périphérie du dispositif de support de sécurité.

Dans la configuration active de retenue, les languettes de retenue 28 et 30 du fourreau intérieur 18 retiennent ce dernier en position escamotée d'attente dans le fourreau de support 16. On pourrait, bien entendu, imaginer que ces languettes soient plutôt formées à la périphérie interne du fourreau extérieur 16 s'accrochant alors sur le fourreau intérieur 18 ou bien que ces languettes élastiques aient un sens d'accrochage inversé par rapport à celui que montre la figure 1 pour venir s'accrocher sur la collerette 20 du corps de seringue 10. Quelle que soit la variante envisagée, si comme c'est avantageusement le cas, on choisit que le piston 12 déclenche le fourreau intérieur 18, ces languettes de retenue 28 et 30 doivent pouvoir être décrochées lorsque le piston 12 de la seringue parvient dans une position de fin de course.

Les extrémités libres respectives 28A et 30A des languettes de retenue 28 et 30 du fourreau intérieur 16 viennent en regard de languettes d'activation 29 et 31 élastiques formées axialement sur le fourreau de support 16 qui sont susceptibles d'être sollicitées vers une configuration inactive dans lesquelles elles autorisent la sortie du fourreau intérieur 18 hors du fourreau de support 16. En l'espèce, ces languettes d'activation 29 et 31 peuvent être sollicitées par une partie de commande du piston 12.

Dans l'exemple représenté, la tête 13 du piston 12 présente cette partie de commande en forme de bride axiale 13A dirigée vers l'avant dont la périphérie interne 13B est inclinée, de telle sorte que, lorsque le piston 12 parvient en fin de course, cette périphérique interne 13B coopère avec les extrémités 29A et 31A des languettes d'activation 29 et 31, et sollicite ces dernières en les resserrant vers l'axe A. En se rapprochant de l'axe A, les languettes d'activation 29 et 31 sollicitent simultanément les portions d'extrémités 28A et 30A de sorte que languettes de retenue 28 et 30 se resserrent à leur tour vers l'axe A et libère leur épaulement 28B et 30B des surfaces de retenue respectives 32 et 34.

En fait, chacune des languettes d'activation 29 et 31 est disposée entre deux des quatre pattes d'encliquetage 24, tandis que chacune des portions axiales 22 est disposée entre les deux autres pattes d'encliquetage 24, de sorte qu'il existe une alternance du type languette d'activation / patte / portion axiale / patte sur toute la périphérie du fourreau de support 16.

Pour simplifier la réalisation du fourreau de support 16, ce dernier comporte comme dans l'exemple représenté, deux portions 16'A et 16'B, la portion 16'A étant de forme relativement simple, tandis que la portion 16B à l'arrière du fourreau de support 16 plus complexe comporte l'ensemble de ces languettes 29, 31, de ces portions axiales 22 et de ces pattes 24. Les deux portions 16'A et 16'B sont maintenues l'une par rapport à l'autre par emmanchement ou tout autre moyen de coopération connu.

En fin de course du piston 12, le fourreau intérieur 18 se sépare du fourreau de support 16 par libération des languettes axiales de retenue 28 et 30 des surfaces de retenue respectives 32 et 34.

Des moyens de poussée, en l'espèce un ressort 36, favorisent la poussée vers l'avant (dans le sens F indiqué sur la figure 1) du fourreau intérieur 18, lorsque les languettes de retenue 28 et 30 sont libérées par pression des languettes d'activation 29 et 31 sollicitées par la tête 13 du piston 12. Ce ressort 36 est logé dans un espace annulaire ménagé entre le corps de seringue 10 et le fourreau intérieur 18.

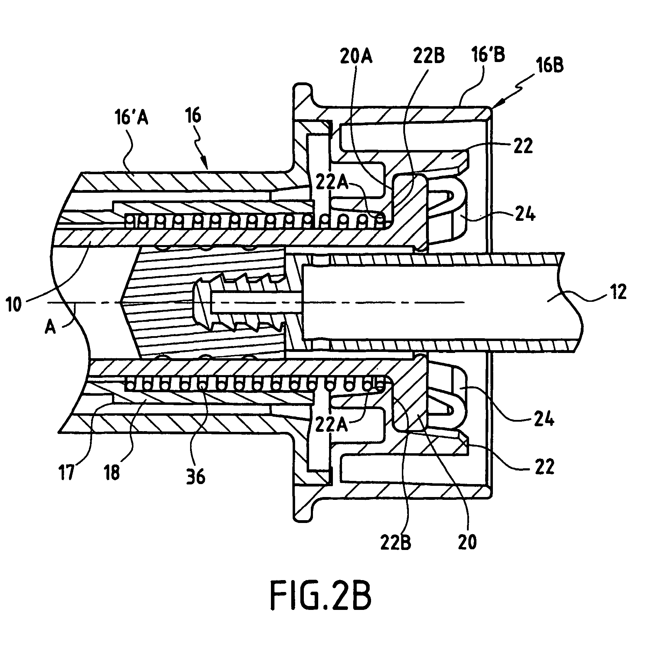

Comme on le voit sur la figure 2B, le fourreau intérieur 18 présente un épaulement 17 formé sur sa périphérie interne en retrait par rapport aux languettes de retenue 28 et 30. Cet épaulement 17 forme une surface d'appui pour le ressort 36. L'extrémité arrière du ressort 36 repose par ailleurs sur une face frontale 22A radiale de chacune des portions de paroi axiale 22.

En fait, les deux portions de paroi axiale 22 portent chacune un secteur radial en saillie vers l'axe A des fourreaux 16 et 18, les faces avant de ces secteurs forment les faces frontales 22A, tandis que leur face arrière forme les épaulements 22B.

Le fourreau intérieur 18 présente en outre une portion tubulaire 19 formée entre l'épaulement 17 et les languettes de retenue 28 et 30 qui s'étendent vers l'arrière du fourreau intérieur 18. Cette portion tubulaire présente un diamètre augmenté par rapport au diamètre du fourreau intérieur 18 et permet d'éviter le contact entre le ressort 36 et les languettes de retenue 28 et 30 et donc tout risque d'endommagement de ces dernières, par frottement par exemple.

L'espace annulaire ménagé pour le ressort 36 est donc limité par la surface d'appui formée par les faces frontales 22A, par l'épaulement 17, par la portion 19 tubulaire, et par les languettes de retenue 28 et 30. En position escamotée d'attente, le ressort 36 est ainsi maintenu précontraint entre ses surfaces d'appui formées d'une part, par les faces frontales 22A et d'autre part, par l'épaulement 17.

Après utilisation de la seringue, et en particulier en fin d'injection, comme illustré sur les figures 3 et 4, le piston 12 est en fin de course et sa tête 13 vient pratiquement en butée contre la collerette 20. Les languettes de retenue 28 et 30 ont été sollicitées par la tête 13, par l'intermédiaire des languettes d'activation 29 et 31, de sorte que les languettes de retenue 28 et 30 se sont décrochées du fourreau de support 16, en l'espèce des surfaces de retenue 32 et 34.

Le fourreau intérieur 18 n'étant plus retenu par les languettes de retenue 28 et 30 dans le fourreau de support 16, le ressort 36 a poussé le fourreau intérieur 18 vers l'avant de telle sorte que ce dernier dépasse largement au-delà de l'extrémité avant 16A du fourreau de support 16, sur une longueur telle qu'il forme une protection autour de l'aiguille 14 sur une longueur suffisante pour éviter les risques de contact d'un utilisateur avec l'aiguille 14.

Dans cette position active de protection, le fourreau intérieur 18 est calé vers l'avant par la venue en butée de son épaulement 17 avec un épaulement 15 formé intérieurement dans une région avant du fourreau extérieur 16. Des moyens de blocage permettent de bloquer le fourreau intérieur 18 dans cette position active de protection. Ces moyens de blocage permettent d'éviter qu'un simple appui sur l'extrémité libre 18A du fourreau intérieur 18 ne permette la rentrée de ce fourreau 18 dans le fourreau extérieur 16, auquel cas l'aiguille 14 pourrait devenir accessible par l'utilisateur.

Ces moyens de blocage comprennent en l'espèce un épaulement 38 formé dans le fourreau de support 16 et orienté à l'opposé de l'épaulement 15. Les extrémités libres 28A et 30A respectives des languettes axiales de retenue 28 et 30 sont aptes à coopérer avec cet épaulement 38 de sorte que le fourreau intérieur 18 se trouve calé entre le premier épaulement 15 l'empêchant d'aller vers l'avant dans le sens F, et le deuxième épaulement 38 l'empêchant de revenir vers l'arrière du dispositif de support de sécurité à l'opposé du déplacement de sortie du fourreau intérieur 18.

Le ressort 36 étant calé entre la face frontale 20A et l'épaulement 17, le dispositif de support de sécurité peut être livré pré-armé avec ou sans seringue. La seringue est alors simplement mise en place dans le dispositif de support de sécurité par simple encliquetage de sa collerette 20 et calage de cette dernière entre les épaulements 22B et les pattes d'encliquetage 24 du fourreau de support 16.

Lorsqu'une seringue est mise en place dans le dispositif de support de sécurité, l'utilisateur manipule l'ensemble constitué par ce dispositif et par cette seringue en tenant le fourreau de support 16, par exemple entre l'index et le majeur, en les calant par exemple sur une collerette 16B formée à l'extrémité arrière de ce fourreau de support 16, et en manipulant le piston 12 par exemple avec le pouce.

La sortie du fourreau intérieur 18 se fait de manière automatique, sans intervention supplémentaire de l'utilisateur dès que le piston 12 atteint sa fin de course. Le choix de la raideur du ressort 36 permet de réguler cette sortie du fourreau intérieur 18 hors du fourreau de support et de limiter l'appui contre la chair du patient.

En outre, pour amortir l'appui dû à la percussion du fourreau intérieur 18 contre la peau du patient, le fourreau intérieur 18 comporte sur sa partie avant un embout 40 souple. L'extrémité libre de cet embout 40 forme l'extrémité libre 18A du fourreau intérieur 18.

Dans l'exemple représenté, le fourreau intérieur 18 est formé par deux parties de rigidité différentes : une première partie rigide 19 par laquelle le fourreau intérieur 18 coopère avec le fourreau de support 16, et une deuxième partie formée par l'embout 40 souple solidaire de la première partie 19.

Classiquement, le fourreau de support 16 et le fourreau intérieur 18 sont tubulaires et réalisés en plastique. Dans l'exemple représenté, l'intégralité de la première partie 19 est rigide, mais le fourreau intérieur 18 pourrait présenter des parties plus ou moins souples comportant des zones rigides qui lui permettent de coulisser sans se déformer dans le fourreau de support 16 et ainsi d'éviter tout risque d'arc-boutement dans ce dernier. Ces zones rigides pourraient, par exemple, être formées par des pattes rectilignes longitudinales qui s'étendraient le long de la première partie 19 du fourreau intérieur 18.

La deuxième partie du fourreau de support formé par l'embout 40 étant quant à elle destinée à amortir l'appui lors de la sortie du fourreau intérieur 18, elle est préférentiellement formée par un matériau plastique choisi parmi le PVC, les polyéthylènes, les élastomères ou le caoutchouc. Le matériau formant l'embout 40 présente avantageusement une dureté Shore comprise entre 30 Hs et 80 Hs, préférentiellement comprise entre 40 Hs et 50 Hs, pour assurer un bon amortissement, même en cas de sortie violente du fourreau intérieur 18, par exemple causé par une défaillance des moyens de retenue.

En outre, dans l'exemple représenté, l'embout 40 présente une épaisseur plus fine vers l'avant. En l'espèce, l'embout 40 présente une première partie 40B sensiblement tubulaire et une partie avant 40A sensiblement tronconique dont l'extrémité la plus fine est tournée vers l'avant du dispositif de sécurité.

Avec une telle conformation de l'embout 40, lors de la sortie du fourreau intérieur 18 hors du fourreau de support 16 et de l'arrivée du fourreau intérieur 18 contre la chair du patient, au moins une partie de l'énergie accumulée par le ressort 36 qui n'aurait pas été libérée par le déplacement du fourreau intérieur 18 dans le fourreau de support 16 serait encaissée par la déformation de l'embout 40.

Selon un premier mode de réalisation, illustré sur la figure 3, cet embout 40 est formé par une pièce additionnelle qui est fixée à la première partie du fourreau intérieur 18 par des moyens de fixation comportant des premiers moyens de fixation présentant au moins un organe en saillie et des deuxièmes moyens de fixation comportant au moins un organe en retrait.

Sur l'exemple représenté sur la figure 3, l'organe en saillie est en l'espèce un bourrelet 42 formé sur la périphérie interne de l'embout 40, et l'organe en retrait est en l'espèce formé par une encoche 44 pratiquée dans la première partie 19 du fourreau intérieur 18. De manière à faciliter la mise en place de l'embout 40, ce dernier présente à son extrémité arrière une ouverture 40C légèrement évasée pour faciliter l'introduction du fourreau intérieur 18. En outre, pour garantir le bon positionnement du fourreau intérieur 18 dans l'embout 40 au moment de l'encliquetage des bourrelets 42 dans les encoches 44, l'embout 40 présente un épaulement intérieur 41, contre lequel l'extrémité libre du fourreau intérieur 18 vient en butée.

On pourrait également imaginer que les bourrelets 42 soient formés sur la première partie 19 du fourreau intérieur 18 et que l'embout 40 présente des encoches 44 destinées à coopérer avec ces bourrelets 42 ou que cette première partie 19 et l'embout 40 présentent chacun un bourrelet et une encoche, le bourrelet de l'un coopérant avec l'encoche de l'autre. Dans ce mode de réalisation, toutes les formes possibles de l'organe en saillie et de l'organe en retrait permettant une coopération de l'embout 40 sur la première partie 19 du fourreau intérieur 18 peuvent être envisagées.

Selon un autre mode de réalisation, l'embout 140 est fixé à la première partie 119 du fourreau intérieur 118, par simple emmanchement en force de l'embout 140 sur l'extrémité libre de la première partie 119, comme illustré sur la figure 4. La pression exercée entre l'embout 140 et la première partie 119 suffit alors à maintenir l'embout 140 sur ce dernier.

Cet embout 140 peut en outre être fixé à la première partie 119 du fourreau intérieur 118 par des moyens 45, choisis parmi le collage, le surmoulage ou le soudage pour assurer son maintien en position.

Dans ce mode de réalisation, l'embout 140 se présente extérieurement de la même manière que l'embout 40 précédemment décrit, avec une partie tronconique 140A identique. De par l'absence de bourrelets, l'intérieur de la partie 140B est simplifiée en une forme cylindrique de diamètre intérieur correspondant au diamètre extérieur de la première partie 119 du fourreau intérieur 118, qui se prolonge jusqu'à l'épaulement 141. Le remplissage de l'ouverture évasée 140C (à l'opposée de l'avant 118A) par un adhésif 45 permet un bon maintien de l'embout 140 sur la première partie 119 du fourreau intérieur 118.

Selon un autre mode de réalisation illustré sur la figure 5, l'embout 240 peut être directement formé par moulage à l'extrémité de la première partie 219 du fourreau intérieur 118, ce dernier formant alors une seule et même pièce. Dans ce cas, deux matériaux, de rigidité différente, sont injectés dans un moule aux zones correspondantes, pour obtenir un fourreau intérieur 218 présentant une partie rigide 219 et une partie souple 240. L'embout 240 présente alors préférentiellement une partie arrière 240B dont la forme permet une bonne liaison entre les matériaux formant l'embout 240 et la première partie 219. La partie avant 218 se présente de manière amincie, comme pour les autres modes de réalisation, avec un contour intérieur 218 apte à coopérer avec un capuchon (non représenté).

Quelle que soit la variante envisagée, l'embout doit être suffisamment souple pour amortir les appuis lors de la percussion du fourreau intérieur contre la chair du patient, mais il est préférable d'éviter, pour des raisons d'hygiène, que l'embout puisse venir en contact avec une partie de l'aiguille. La déformation de l'embout ne doit pas pouvoir permettre à ce dernier de toucher l'aiguille, même lorsqu'une pression importante, due par exemple à un écrasement de l'embout, est exercée sur ce dernier. On pourra par exemple, selon la dureté du matériau, être amené à positionner l'extrémité libre de l'embout au ras de l'extrémité du corps de seringue proche de l'aiguille, en position escamotée d'attente.

L'ensemble formé par le dispositif de support de sécurité et par la seringue comporte en outre un capuchon amovible qui protège l'aiguille avant utilisation et en particulier lorsque le fourreau intérieur 18 est en position escamotée d'attente. Ce capuchon protège l'aiguille 14 de la seringue et coopère avec le corps de seringue 18 sur sa partie avant 10A. En l'espèce, le capuchon 46 représenté sur la figure 1 est en deux parties 46 et 47, disposées l'une autour de l'autre.

Dans la position escamotée d'attente du fourreau intérieur 18, l'embout 40 entoure sensiblement sans jeu le capuchon 46 de manière à protéger la seringue et l'aiguille 14 de toute contamination extérieure par liaison sensiblement étanche. En l'espèce, l'embout 40 présente un contour intérieur 48 de géométrie sensiblement identique à celle du contour extérieur du capuchon 46. Le capuchon 46 est fréquemment de forme tronconique évasée vers son ouverture, de sorte qu'il suffit de choisir un contour intérieur 48 de l'embout 40 de diamètre sensiblement égal au diamètre moyen du contour extérieur 40 du capuchon 46. Ainsi, l'embout 40 vient coopérer avec le capuchon 46 par légère déformation sur de l'embout 40 qui épouse le contour du capuchon 46. Le capuchon 46 amovible est retiré par l'utilisateur avant injection, en tirant simplement le capuchon 46 avec une main par exemple, tandis que l'autre main de l'utilisateur retient le dispositif de support de sécurité.

## Revendications

1. Dispositif de support de sécurité pour une seringue, comprenant un fourreau de support (16) pour le corps (20) d'une seringue et des moyens de sécurité qui comprennent un fourreau intérieur (18 ; 118) susceptible de coulisser par rapport au dit fourreau de support (16), entre une position escamotée d'attente, dans laquelle ledit fourreau intérieur (18 ; 118 ; 218) est sensiblement escamoté à l'intérieur dudit fourreau de support (16), et une position active de protection, dans laquelle ledit fourreau intérieur (18 118 ; 218) dépasse dudit fourreau de support (16), le fourreau intérieur étant sollicité dans cette position active de protection par des moyens de poussée (36),
**caractérisé en ce que** ledit fourreau intérieur (18 ; 118 ; 218) présente une portion d'extrémité libre formée par un embout (40 ; 140 ; 240) souple et **en ce que** ledit embout (40 ; 140 ; 240) présente une extrémité libre amincie (40A ; 140A ; 240A).

2. Dispositif selon la revendication 1, **caractérisé en ce que** ledit fourreau intérieur (18 ; 118 ; 218) comporte une première partie rigide (19 ; 119 ; 219) par laquelle ledit fourreau intérieur (18 ; 118 ; 218) coopère avec ledit fourreau de support (16), et une deuxième partie (40 ; 140 ; 240) formée par ledit embout (40 ; 140 ; 240) souple qui est solidaire de ladite première partie (19 ; 119 ; 219).

3. Dispositif selon la revendication 2, **caractérisé en ce que** ledit embout (140) est fixé à ladite première partie (119) par des moyens (45) choisis parmi le collage, le surmoulage, le soudage ou l'emmanchement en force.

4. Dispositif selon la revendication 2 ou 3, **caractérisé en ce que** ledit embout (40) est fixé à ladite première partie (19) par des moyens comprenant des premiers moyens de fixation comportant un organe en saillie (42) et appartenant à l'un (19 ; 40) des deux éléments constitués par ladite première partie (19) et ledit embout (40), et des deuxièmes moyens de fixation comportant un organe en retrait (44) appartenant à l'autre de ces deux éléments (19 ; 40).

5. Dispositif selon l'une quelconque des revendications précédentes, **caractérisé en ce que** le matériau dudit embout (40 ; 140 ; 240) présente une dureté Shore comprise entre 30 Hs et 80 Hs.

6. Dispositif selon l'une quelconque des revendications précédentes, **caractérisé en ce qu'**il comporte en outre des moyens de retenue (28, 30, 32, 34) susceptibles d'adopter une configuration active de retenue dans laquelle ils retiennent ledit fourreau intérieur (18 ; 118 ; 218) en position escamotée d'attente et une configuration inactive dans laquelle ils autorisent la sortie dudit fourreau intérieur (18 ; 118 ; 218) sous l'action de moyens de poussée (36).

7. Dispositif selon la revendication précédente, **caractérisé en ce que** lesdits moyens de retenue (28, 30, 32, 34) comportent des languettes de retenue élastiques (28, 30) et des surfaces de retenue (32, 34), lesdites languettes de retenue (28; 30) ou lesdites surfaces de retenue (32, 34) étant solidaires dudit fourreau intérieur (18 ; 118 ; 218), lesdites languettes de retenue (28, 30) étant retenues sur les surfaces de retenue (32, 34) en configuration active de retenue et étant susceptibles d'être déplacées élastiquement vers leur configuration inactive dans laquelle lesdites languettes de retenue (28, 30) échappent aux dites surfaces de retenue (32, 34).

8. Ensemble d'un dispositif selon l'une quelconque des revendications précédentes et d'une seringue ayant un corps (20), un piston (12) et une aiguille (14) disposée à une extrémité dudit corps (20).

9. Ensemble selon la revendication précédente et la revendication 6 ou 7, **caractérisé en ce que** ledit corps de seringue (20) est maintenu fixement dans ledit fourreau de support (16) et **en ce que** ledit piston (12) de la seringue comporte une partie de commande (13, 13A) apte à commander lesdits moyens de retenue (28, 30, 32, 34) vers leur configuration inactive.

10. Ensemble selon la revendication 7 et la revendication 8 ou 9, **caractérisé en ce que** lesdites languettes de retenue (28, 30) sont situées au voisinage d'une extrémité (16B) dudit fourreau de support (16) opposée à l'aiguille (14) et **en ce que** ledit piston (12) présente une tête (13) apte à solliciter lesdites languettes de retenue (28, 30) vers leur configuration inactive.

11. Ensemble selon l'une quelconque des revendications 8 à 10, **caractérisé en ce qu'**il comporte en outre un capuchon (46, 47) amovible recouvrant l'aiguille (14) et **en ce qu'**en position escamotée dudit fourreau intérieur (18), ledit embout (40) entoure sensiblement sans jeu ledit capuchon (46).

## Claims

1. A safe support device for a syringe, said safe support device comprising a support sheath (16) for the body (20) of a syringe, and safety means which comprise an inner sheath (18; 118) suitable for sliding relative to said support sheath (16) between a waiting retracted position, in which said inner sheath (18; 118; 218) is substantially retracted inside said support sheath (16), and an active protection position, in which said inner sheath (18; 118; 218) projects from said support sheath (16), the inner sheath being driven into this active protection position by drive means (36),
said safe support device being **characterized in that** said inner sheath (18; 118; 218) has a free end portion formed by a flexible end-piece (40; 140; 240) and **in that** said end-piece (40; 140; 240) has a tapering free end (40A; 140A; 240A).

2. A device according to claim 1, **characterized in that** said inner sheath (18; 118; 218) has a rigid first portion (19; 119, 219) via which said inner sheath (18; 118, 218) co-operates with said support sheath (16), and a second portion (40; 140; 240) formed by said flexible end-piece (40; 140; 240) which is secured to said first portion (19; 119; 219).

3. A device according to claim 2, **characterized in that** said end-piece (140) is fixed to said first portion (119) by means (45) chosen from bonding with an adhesive, overmolding, heat-sealing, or force fitting.

4. A device according to claim 2 or claim 3, **characterized in that** said end-piece (40) is fixed to said first portion (19) by means made up of first fixing means comprising a projecting member (42) that is part of one of the two elements (19; 40) constituted by said first portion (19) and by said end-piece (40), and of second fixing means comprising a set-back member (44) that is part of the other of the two elements (19; 40).

5. A device according to any preceding claim, **characterized in that** the material of said end-piece (40; 140; 240) has Shore hardness lying in the range 30 Hs to 80 Hs.

6. A device according to any preceding claim, **characterized in that** it further comprises retaining means (28, 30, 32, 34) suitable for taking up a retaining active configuration in which they retain said inner sheath (18; 118; 218) in the waiting retracted position, and an inactive configuration in which they allow said inner sheath (18; 118; 218) to be deployed under the action of drive means (36).

7. A device according to the preceding claim, **characterized in that** said retaining means (28, 30, 32, 34) comprise resilient retaining tongues (28, 30) and retaining surfaces (32, 34), said retaining tongues (28, 30) or said retaining surfaces (32, 34) being integral with said inner sheath (18; 118; 218), said retaining tongues (28, 30) being retained on the retaining surfaces (32, 34) in the active retaining configuration and being suitable for being moved resiliently towards their inactive configuration, in which said retaining tongues (28, 30) are released from said retaining surfaces (32, 34).

8. An assembly comprising a device according to any preceding claim, and a syringe having a body (20), a piston (12) and a needle (14) disposed at one end of said body (20).

9. An assembly according to the preceding claim, and to claim 6 or claim 7, **characterized in that** said syringe body (20) is held securely inside said support sheath (16) and **in that** said piston (12) of the syringe has a control portion (13, 13A) suitable for causing said retaining means (28, 30, 32, 34) to move into their inactive configuration.

10. An assembly according to claim 7, and to claim 8 or claim 9, **characterized in that** said retaining tongues (28, 30) are situated in the vicinity of one end (16B) of said support sheath (16), which end is remote from the needle (14), and **in that** said piston (12) has a head (13) suitable for driving said retaining tongues (28, 30) into their inactive configuration.

11. An assembly according to any one of claims 8 to 10, **characterized in that** it further comprises a removable cap (46, 47) covering the needle (14), and **in that**, when said inner sheath (18) is in the retracted position, said end-piece (40) surrounds said cap (46) substantially snugly.

## Patentansprüche

1. Sicherheitstragvorrichtung für eine Spritze, umfassend eine Traghülse (16) für den Körper (20) einer Spritze sowie Sicherheitsmittel, die eine Innenhülse (18; 118) umfassen, welche gegenüber der Traghülse (16) zwischen einer eingezogenen Wartestellung, in der die Innenhülse (18; 118; 218) im wesentlichen in die Traghülse (16) eingezogen ist, und einer aktiven Schutzstellung, in der die Innenhülse (18; 118; 218) über die Traghülse (16) hinausragt, verschieblich ist, wobei die Innenhülse in dieser aktiven Schutzstellung durch Schubmittel (36) belastet ist,
**dadurch gekennzeichnet, daß** die Innenhülse (18; 118; 218) einen freien Endabschnitt aufweist, der von einem flexiblen Ansatzstück (40; 140; 240) gebildet ist, und daß das Ansatzstück (40; 140; 240) ein verjüngtes freies Ende (40A; 140A, 240A) aufweist.

2. Vorrichtung nach Anspruch 1, **dadurch gekennzeichnet, daß** die Innenhülse (18; 118; 218) einen ersten starren Teil (19; 119; 219) umfaßt, über den die Innenhülse (18; 118; 218) mit der Traghülse (16) zusammenwirkt, sowie einen von dem flexiblen Ansatzstück (40; 140; 240) gebildeten zweiten Teil (40; 140; 240), der mit dem ersten Teil (19; 119; 219) fest verbunden ist.

3. Vorrichtung nach Anspruch 2, **dadurch gekennzeichnet, daß** das Ansatzstück (140) durch Mittel (45), die aus Kleben, Anformen, Schweißen oder Aufpressen ausgewählt sind, an dem ersten Teil (119) befestigt ist.

4. Vorrichtung nach Anspruch 2 oder 3, **dadurch gekennzeichnet, daß** das Ansatzstück (40) an dem ersten Teil (19) durch Mittel befestigt ist, die erste Befestigungsmittel umfassen, welche ein vorspringendes Organ (42) aufweisen und zu einem (19; 40) der beiden Elemente, die von dem ersten Teil (19) und dem Ansatzstück (40) gebildet sind, gehören, sowie zweite Befestigungsmittel umfassen, welche ein zurückspringendes Organ (44) aufweisen, das zu dem anderen dieser beiden Elemente (19; 40) gehört.

5. Vorrichtung nach einem der vorhergehenden Ansprüche, **dadurch gekennzeichnet, daß** das Material des Ansatzstücks (40; 140; 240) eine Shore-Härte im Bereich zwischen 30 Hs und 80 Hs aufweist.

6. Vorrichtung nach einem der vorhergehenden Ansprüche, **dadurch gekennzeichnet, daß** sie ferner Haltemittel (28, 30, 32, 34) umfaßt, die geeignet sind, eine aktive Halteanordnung, in der sie die Innenhülse (18; 118; 218) in der eingezogenen Wartestellung halten, sowie eine inaktive Anordnung einzunehmen, in der sie das Ausfahren der Innenhülse (18; 118; 218) unter der Wirkung von Schubmitteln (36) zulassen.

7. Vorrichtung nach dem vorhergehenden Anspruch, **dadurch gekennzeichnet, daß** die Haltemittel (28, 30, 32, 34) elastische Haltezungen (28, 30) und Halteflächen (32, 34) umfassen, wobei die Haltezungen (28, 30) oder die Halteflächen (32, 34) mit der Innenhülse (18; 118; 218) fest verbunden sind, wobei die Haltezungen (28, 30) in der aktiven Halteanordnung an den Halteflächen (32, 34) gehalten werden und geeignet sind, elastisch in ihre inaktive Anordnung bewegt zu werden, in der die Haltezungen (28, 30) von den Halteflächen (32, 34) freikommen.

8. Anordnung aus einer Vorrichtung nach einem der vorhergehenden Ansprüche und aus einer Spritze, die einen Körper (20), einen Kolben (12) und eine an einem Ende des Körpers (20) angeordnete Nadel (14) aufweist.

9. Anordnung nach dem vorhergehenden Anspruch und Anspruch 6 oder 7, **dadurch gekennzeichnet, daß** der Spritzenkörper (20) in der Traghülse (16) fest gehalten ist und daß der Kolben (12) der Spritze einen Betätigungsteil (13, 13A) aufweist, der geeignet ist, die Haltemittel (28, 30, 32, 34) in ihre inaktive Anordnung zu betätigen.

10. Anordnung nach Anspruch 7 und Anspruch 8 oder 9, **dadurch gekennzeichnet, daß** sich die Haltezungen (28, 30) in der Nähe eines der Nadel (14) gegenüberliegenden Endes (16B) der Traghülse (16) befinden und daß der Kolben (12) einen Kopf (13) aufweist, der geeignet ist, die Haltezungen (28, 30) in ihre inaktive Anordnung zu belasten.

11. Anordnung nach einem der Ansprüche 8 bis 10, **dadurch gekennzeichnet, daß** sie ferner eine abnehmbare, die Nadel (14) bedeckende Kappe (46, 47) umfaßt und daß in der eingezogenen Position der Innenhülse (18) das Ansatzstück (40) die Kappe (46) im wesentlichen spielfrei umgibt.
